# EUROPEAN PATENT SPECIFICATION

(11) **EP 2 588 472 B1**
(45) Date of publication and mention of the grant of the patent: **09.04.2014**
(21) Application number: 11728293.9
(22) Date of filing: 30.06.2011
(51) Int. Cl.: C07D 401/14

(54) **RUPATADINE derivative AS AN ANTIHISTAMINIC AGENT**
RUPATADINderivat ALS ANTIHISTAMINIKUM
Derivé DE RUPATADINE UTILISÉ EN TANT QU'AGENT ANTIHISTAMINIQUE

(30) Priority: 16.07.2010 US 364985 P; 30.06.2010 EP 10382183
(43) Date of publication of application: 08.05.2013
(73) Proprietor: J. Uriach y Compania S.A., 08184 Palau-solità i Plegamans (Barcelona) (ES)
(72) Inventor: CARCELLER GONZÁLEZ, Elena, E-08190 Sant Cugat del Vallès - Barcelona (ES); BELLOC PASCUAL, Jordi, E-08027 Barcelona (ES)
(74) Representative: ABG Patentes, S.L.
(86) International application number: PCT/EP2011/061035
(87) International publication number: WO 2012/001120

(56) References cited:
- EP-A1- 0 577 957
- MANUEL MERLOS ET AL: "Rupatadine, a New Potent, Orally Active Dual Antagonist of Histamine and Platelet-Activating Factor (PAF)", THE JOURNAL OF PHARMACOLOGY AND EXPERIMENTAL THERAPEUTICS, THE AMERICAN SOCIETY FOR PHARMACOLOGY AND EXPERIMENTAL THERAPEUTICS, vol. 280, no. 1, 1 January 1997 (1997-01-01), pages 114-121, XP007919175, DOI: DOI:0022-3565/97/2801-0114 [retrieved on 1997-01-01]
- GOULD P L: "SALT SELECTION FOR BASIC DRUGS", INTERNATIONAL JOURNAL OF PHARMACEUTICS, ELSEVIER BV, NL, vol. 33, no. 1/03, 1 January 1986 (1986-01-01), pages 201-217, XP002074725, ISSN: 0378-5173, DOI: DOI:10.1016/0378-5173(86)90055-4

## Description

### Field of the invention

The present application relates to a novel antihistaminic agent and pharmaceutical compositions thereof.

### Background of the invention

Rupatadine (I) is an authorized antihistaminic agent and has IUPAC name 8-Chloro-6,11-dihydro-11-[1-[(5-methyl-3-pyridinyl)methyl]-4-piperidinylidene]-5*H*-benzo[5,6]cyclohepta[1,2-b]pyridine, CAS number 158876-82-5 for the free base and the following chemical formula:

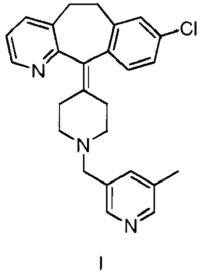

Rupatadine is disclosed in EP0577957. Rupatadine is currently marketed in 10 mg tablets as rupatadine fumarate for the treatment of allergic rhinitis and urticaria in adults and teenagers. Rupatadine fumarate was first disclosed in Spanish patent application ES2087818.

It is always desirable to provide new compounds that inhibit the histamine H1 receptor, *i.e*. antihistaminic agents. Furthermore it is always desirable to provide new compounds with properties that make them suitable to different pharmaceutical dosage forms.

Rupatadine fumarate has low solubility in water (2.9 mg/mL) and it is desirable to provide antihistaminic agents with high solubility in order to easily prepare liquid formulations for the treatment of diseases that can be treated with an antihistaminic agent.

### Brief description of the invention

The present invention is directed to a compound of structural formula **II**: 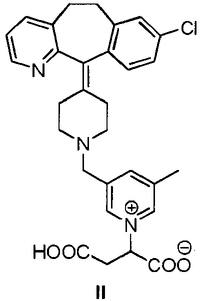 or a pharmaceutically acceptable salt thereof.

Another embodiment of the present invention is a compound of formula **II** or a pharmaceutically acceptable salt thereof for use in therapy.

Another embodiment of the present invention is a compound of formula **II** or a pharmaceutically acceptable salt thereof for use in the treatment of an allergic disorder.

Another embodiment of the present invention is a compound of formula **II** or a pharmaceutically acceptable salt thereof for use in the treatment of allergic rhinitis or urticaria.

Another embodiment of the present invention is a pharmaceutical composition comprising the compound of formula **II** or a pharmaceutically acceptable salt thereof and one or more pharmaceutically acceptable excipients.

Another embodiment of the present invention is a method of treating an allergic disorder which comprises administering to a subject in need thereof an amount of a compound of formula **II** or a pharmaceutically acceptable salt thereof effective to treat said disorder.

Another embodiment of the present invention is the use of compound of formula **II** or a pharmaceutically acceptable salt thereof for the treatment of an allergic disorder.

A further embodiment of the present invention is a process for the preparation of the compound of formula **II** which comprises reacting a compound of formula **I** with a compound of formula **III**, wherein LG is a leaving group. In a preferred embodiment, the reaction is conducted in a solvent:

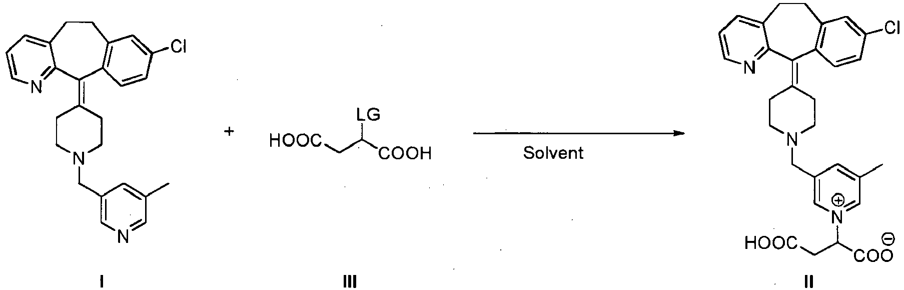

A further embodiment of the present invention is a process for the preparation of the compound of formula **II** which comprises reacting a compound of formula **I** with fumaric acid in acidic conditions:

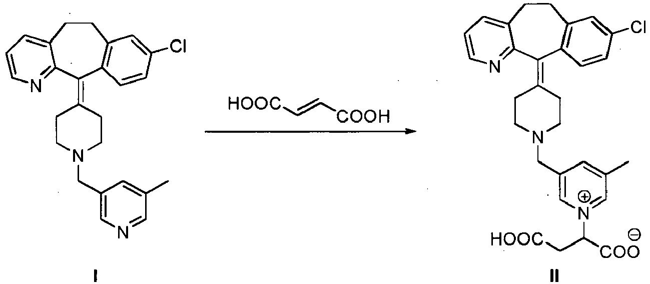

### Detailed description of the invention

The invention relates to the compound of formula **II** or a pharmaceutically acceptable salt thereof. Compound of formula **II** is an antihistaminic agent (see Example 2).

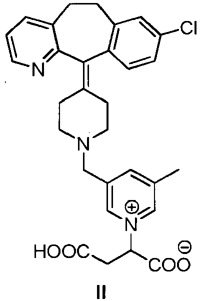

Compound **II** can be present in a tautomeric form, *i.e.* with a hydrogen atom in a different position as depicted below. All tautomeric forms of the compound of formula **II** are included herein.

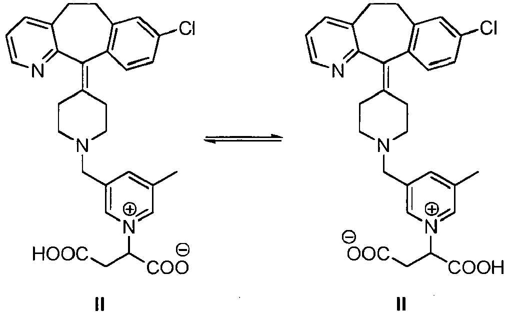

Furthermore, the inventors have also found that compound **II** shows a high solubility in water (>50 g/L), which is a very desirable feature for a drug because it allows the easy preparation of aqueous liquid formulations.

Being an antihistaminic agent, compound **II** is useful for the treatment of diseases mediated by histamine such as allergic disorders (e.g. allergic rhinitis, conjunctivitis, pruritus, urticaria, dermatitis), asthma and anaphylactic shock.

Another embodiment of the present invention is a compound of formula **II** or a pharmaceutically acceptable salt thereof for use in therapy.

Another embodiment of the present invention is a compound of formula **II** or a pharmaceutically acceptable salt thereof for use in the treatment of an allergic disorder. In another embodiment, the allergic disorder is allergic rhinitis or urticaria.

Another embodiment of the present invention is a method of treating an allergic disorder which comprises administering to a subject in need thereof, particularly a human being, an amount of a compound of formula **II** or a pharmaceutically acceptable salt thereof effective to treat said disorder. In another embodiment the allergic disorder is urticaria or allergic rhinitis.

Another embodiment of the present invention is the use of compound of formula **II** or a pharmaceutically acceptable salt thereof for the treatment of an allergic disorder. In another embodiment, the allergic disorder is allergic rhinitis or urticaria.

Throughout the present specification, by the term "treatment" or "treating" is meant eliminating, reducing or ameliorating the cause or the effects of a disorder. For purposes of this invention treatment includes, but is not limited to, alleviation, amelioration or elimination of one or more symptoms of the disorder; diminishment of the extent of the disorder; stabilized (i.e. not worsening) state of the disorder; delay or slowing of disorder progression; amelioration or palliation of the disorder state; and remission of the disorder (whether partial or total).

The term "pharmaceutically acceptable salt" refers to those salts which are, according to medical judgement, suitable for use in contact with the tissues of humans and other mammals without undue toxicity, irritation, allergic response and the like. Pharmaceutically acceptable salts are well known in the art.

Another embodiment of the present invention is a pharmaceutical composition comprising the compound of formula **II** or a pharmaceutically acceptable salt thereof, and one or more pharmaceutically acceptable excipients. The excipients must be "acceptable" in the sense of being compatible with the other ingredients of the composition and not deleterious to the recipients thereof. Pharmaceutically acceptable excipients are well known in the art. See e.g. Handbook of pharmaceutical excipients, APhA Publications 5th edition 2005, edited by Raymond C. Rowe, Paul J. Sheskey, Siân C. Owen, ISBN-10: 1582120587.

The compound of the present invention can be administered in the form of any pharmaceutical formulation, the nature of which, as it is well known, will depend upon the nature of the active compound and its route of administration. Any route of administration may be used, for example oral, parenteral, nasal, ocular, and topical administration.

Solid compositions for oral administration include tablets, granulates and capsules. In any case the manufacturing method is based on a simple mixture, dry granulation or wet granulation of the active compound with excipients. These excipients can be, for example, diluents such as lactose, microcrystalline cellulose, mannitol or calcium hydrogenphosphate; binding agents such as for example starch, gelatin or povidone; disintegrants such as sodium carboxymethyl starch or sodium croscarmellose; and lubricating agents such as for example magnesium stearate, stearic acid or talc. Tablets can be additionally coated with suitable excipients by using known techniques with the purpose of delaying their disintegration and absorption in the gastrointestinal tract and thereby provide a sustained action over a longer period, or simply to improve their organoleptic properties or their stability. The active compound can also be incorporated by coating onto inert pellets using natural or synthetic film-coating agents. Soft gelatin capsules are also possible, in which the active compound is mixed with water or an oily medium, for example coconut oil, mineral oil or olive oil.

Powders and granulates for the preparation of oral suspensions by the addition of water can be obtained by mixing the active compound with dispersing or wetting agents; suspending agents and preservatives. Other excipients can also be added, for example sweetening, flavoring and colouring agents.

Liquid forms for oral administration include emulsions, solutions, suspensions, syrups and elixirs containing commonly used inert diluents, such as purified water, ethanol, sorbitol, glycerol, polyethylene glycols (macrogols) and propylene glycol. Said compositions can also contain coadjuvants such as wetting, suspending, sweetening, flavoring agents, preservatives and buffers.

Injectable preparations, according to the present invention, for parenteral administration, comprise sterile solutions, suspensions or emulsions, in an aqueous or non-aqueous solvent such as propylene glycol, polyethylene glycol or vegetable oils. These compositions can also contain coadjuvants, such as wetting, emulsifying, dispersing agents and preservatives. They may be sterilized by any known method or prepared as sterile solid compositions, which will be dissolved in water or any other sterile injectable medium immediately before use. It is also possible to start from sterile materials and keep them under these conditions throughout all the manufacturing process.

The compound of the invention can also be formulated for its topical application for the treatment of pathologies occurring in zones or organs accessible through this route, such as eyes, skin and the intestinal tract. Formulations include creams, lotions, gels, powders, solutions and patches wherein the compound is dispersed or dissolved in suitable excipients.

For the nasal administration or for inhalation, the compound can be formulated as an aerosol and it can be conveniently released using suitable propellants.

The dosage and frequency of doses will depend upon the nature and severity of the disease to be treated, the age, the general condition and body weight of the patient, as well as the the route of administration, among other factors. A representative example of a suitable dosage range is from about 0.01 mg/kg to about 100 mg/kg per day, which can be administered as a single or divided doses.

Another embodiment of the present invention is a liquid aqueous pharmaceutical composition comprising the compound of formula **II** or a pharmaceutically acceptable salt thereof. In an embodiment said aqueous liquid pharmaceutical composition comprises from 0.01 g/L to 10 g/L of the compound of formula **II**. In another embodiment the aqueous liquid pharmaceutical composition is intended for oral use.

Another embodiment of the present invention is a pharmaceutical composition comprising a compound of formula **II** or a pharmaceutically acceptable salt thereof which further comprises a second antihistaminic agent. An antihistaminic agent is a compound which inhibits the histamine H1 receptor. Any antihistaminic agent known in the literature can be used as second antihistaminic agent. In a preferred embodiment, the second antihistaminic agent is selected from loratadine, desloratadine, rupatadine, clemastine, diphenhydramine, fexofenadine, pheniramine, cetirizine, ebastine, promethazine, chlorpheniramine and levocetirizine, or a pharmaceutically acceptable salt thereof. In a further preferred embodiment, the second antihistaminic agent is rupatadine or a pharmaceutically acceptable salt thereof, particularly rupatadine fumarate.

A further embodiment of the present invention is a process for the preparation of the compound of formula **II** which comprises reacting a compound of formula **I** with a compound of formula III, wherein LG is a leaving group,in a solvent :

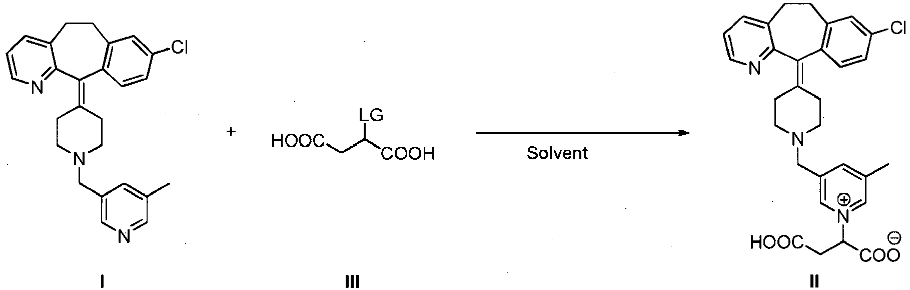

Preferably, the solvent is selected from alcohol, water, mixtures of alcohol and water, and mixtures of water and a polar aprotic solvent. A polar aprotic solvent is a solvent which shows a separation of charge in its molecule and that can not exchange a hydrogen ion with another compound. Any polar aprotic solvent can be used. In a preferred embodiment, the polar aprotic solvent is acetonitrile, DMSO, DMF, dioxane, THF, acetone and the like. Any liquid alcohol at the temperature of the reaction can be used. In an embodiment the alcohol is methanol, ethanol, propanol, isopropanol, butanol, pentanol, hexanol and the like. In an embodiment the reaction takes place using an alcohol as solvent. The reaction can take place at a temperature from room temperature to the boiling point of the solvent.

As indicated above, LG is a leaving group. A leaving group is a part of the molecule which in a heterolytic bond cleavage keeps two electrons. Any leaving group disclosed in the literature can in principle be used. Suitable leaving groups include halogen atoms (Cl, Br or I); -O-SO₂R¹, wherein R¹ is CF₃, methyl, tolyl or F, and the like. Further leaving groups are disclosed in Smith, March. Advanced Organic Chemistry 6th ed. (pg. 501-502).

A further embodiment of the present invention is a process for the preparation of the compound of formula **II** which comprises reacting a compound of formula **I** with fumaric acid in acidic conditions:

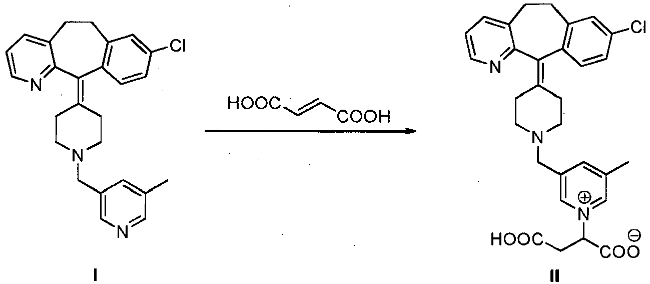

The following examples illustrate the scope of the invention.

### Examples

### Example 1: Preparation of compound II

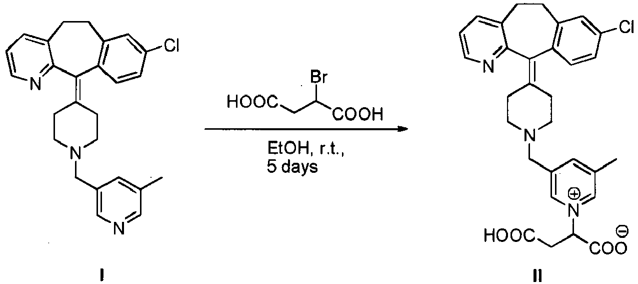

35 g (0.084 mol) of rupatadine (**I**) were dissolved in 260 mL of ethanol in a 1000 mL flask. To that solution 16.1 g (0.087 mol) of bromosuccinic acid were added and the mixture was allowed to react overnight at room temperature. The mixture was concentrated to half the volume and allowed to react at room temperature during 5 days.

The solvent was removed in vacuo and the solid washed with ethanol and with an ethanol:water:ammonia mixture. Next, it was purified by flash chromatography using a 9:1:1 ethanol:ammonia:water mixture.

Finally 4.51 g (0.0085 mol, 9.7 % yield) of the desired product (**II**) were obtained.

**¹H-NMR** (300 MHz, CD₃OD): 8.71 (wide signal, 2H), 8.31 (m, 2H), 7.63 (d, *J*=7.31 Hz, 1H), 7.18 (m, 4H), 5.48 (dd, *J*=3.29 Hz, *J*=11.70 Hz, 1H), 3.69 (s, 2H), 3.41 (m, 3H), 3.11 (dd, *J*=11.7 Hz, *J*=17.2 Hz, 1 H), 2.8 (m, 4H), 2.54 (s, 3H), 2.45 (m, 2H), 2.26 (m, 4H).

**¹³C-NMR** (75.43 MHz, CD₃OD): 176.35, 172.20, 158.73, 147.00, 144.65, 143.31, 143.26, 141.15, 140.03, 139.69, 139.47, 138.65, 138.62, 135.85, 134.06, 133.72, 131.91, 130.33, 127.01, 124.08, 75.81, 59.36, 55.47, 55.42, 42.30, 32.75, 32.03, 31.81, 31.77, 18.38.

**IR** (KBr): 3412, 1587, 1474, 1437, 1379, 1176, 1086, 992, 874, 829, 663 cm⁻¹.

| | | |
|---|---|---|
| **EA:** | Calculated (C₃₀H₃₀ClN₃O₄·NH₃·H₂O): | C 63.54; H 6.22; N 9.88 |
| | Found: | C 63.47; H 6.41; N 9.61 |

### Example 2: Measurement of the histamine H1 receptor inhibitory capacity of compound II

The antiH1 activity of Compound II was assayed using a standard H1 binding assay. The test was performed following a protocol based on Smit *et al.* (*Brit. J. Pharmacol.* **1996** *117(6)*, 1071-1080): the binding was performed on recombinant HEK-293 cells expressing the human H1 receptor. The reference compound was [³H]-pyrilamine at 1 nM. Recombinant HEK-293 derived membranes were incubated at 22°C for 60 minutes with [³H]-pyrilamine in the presence of different concentrations of compound II. Measurements were carried out using scintillation counting.

Compound II exhibited an IC50 value of 0.37 µM in this assay.

### Example 3: Liquid formulations of Compound II

| **Component** | Amount |
|---|---|
| Compound II | 0.200 g |
| Propylene glycol | 20.00 g |
| Sucrose | 30.00 g |
| Saccharin sodium | 0.050 g |
| Methyl parahydroxybenzoate | 0.100 g |
| Colorant | 0.0001 g |
| Flavouring agent | 0.250 g |
| Purified water qs | 100 mL |

Said formulation is prepared by dissolving Compound II in propylene glycol; this is the "active solution". The other excipients are dissolved in water, this is the "excipients solution". When both solutions are completely dissolved, they are mixed and further water is added to the desired volume.

## Claims

1. A compound of formula **II**: 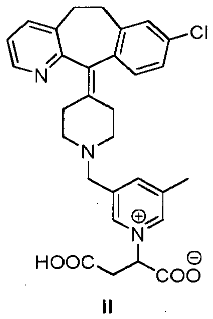 or a pharmaceutically acceptable salt thereof.

2. A compound according to claim 1 for use in therapy.

3. A compound according to claim 2 for use in the treatment of an allergic disorder.

4. A compound according to claim 2 for use in the treatment of allergic rhinitis or urticaria.

5. A pharmaceutical composition comprising the compound of claim 1 and one or more pharmaceutically acceptable excipients.

6. The pharmaceutical composition according to claim 5 which is a liquid aqueous pharmaceutical composition.

7. The pharmaceutical composition according to claim 6 which comprises from 0.01 g/L to 10 g/L of the compound according to claim 1.

8. The pharmaceutical composition according to any of claims 5 to 7 which is intended for oral use.

9. The pharmaceutical composition according to any of claims 5 to 8 which comprises a second antihistaminic agent.

10. The pharmaceutical composition according to claim 9 wherein the second antihistaminic agent is selected from loratadine, desloratadine, rupatadine, clemastine, diphenhydramine, fexofenadine, pheniramine, cetirizine, ebastine, promethazine, chlorpheniramine and levocetirizine, or a pharmaceutically acceptable salt thereof.

11. The pharmaceutical composition according to claim 10 wherein the second antihistaminic agent is rupatadine or a pharmaceutically acceptable salt thereof.

12. A process for the preparation of the compound according to claim 1 which comprises reacting a compound of formula **I** with a compound of formula **III** wherein LG is a leaving group, in a solvent

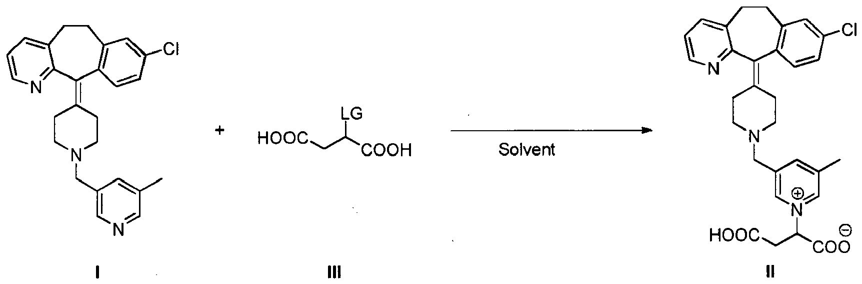

13. The process according to claim 12 wherein the solvent is selected from an alcohol, water, mixtures of an alcohol and water and mixtures of water and a polar aprotic solvent.

14. The process according to claim 13 wherein the solvent is an alcohol.

15. A process for the preparation of the compound according to claim 1 which comprises reacting a compound of formula **I** with fumaric acid in acidic conditions:

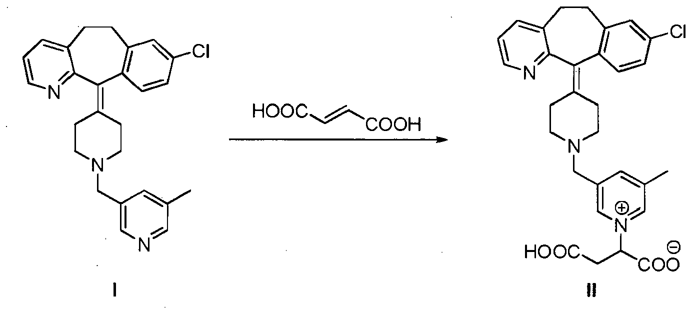

## Patentansprüche

1. Verbindung der Formel II: 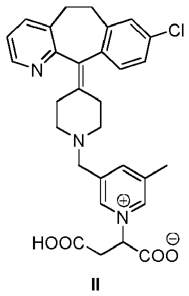 oder ein pharmazeutisch annehmbares Salz davon.

2. Verbindung nach Anspruch 2 zur Verwendung in einer Therapie.

3. Verbindung nach Anspruch 2 zur Verwendung in der Behandlung einer allergischen Erkrankung.

4. Verbindung nach Anspruch 2 zur Verwendung in der Behandlung von allergischer Rhinitis oder Urtikaria.

5. Pharmazeutische Zusammensetzung, enthaltend die Verbindung nach Anspruch 1 und einen oder mehrere pharmazeutisch annehmbare Hilfsstoffe.

6. Pharmazeutische Zusammensetzung nach Anspruch 5, die eine flüssige wässrige pharmazeutische Zusammensetzung ist.

7. Pharmazeutische Zusammensetzung nach Anspruch 6, die 0,01 g/l bis 10 g/l der Verbindung nach Anspruch 1 enthält.

8. Pharmazeutische Zusammensetzung nach einem der Ansprüche 5 bis 7, die für eine orale Verwendung bestimmt ist.

9. Pharmazeutische Zusammensetzung nach einem der Ansprüche 5 bis 8, die ein zweites Antihistaminikum enthält.

10. Pharmazeutische Zusammensetzung nach Anspruch 9, wobei das zweite Antihistaminikum aus Loratadin, Desloratadin, Rupatadin, Clemastin, Diphenhydramin, Fexofenadin, Pheniramin, Cetirizin, Ebastin, Promethazin, Chlorpheniramin und Levocetirizin, oder einem pharmazeutisch annehmbaren Salz davon ausgewählt wird.

11. Pharmazeutische Zusammensetzung nach Anspruch 10, wobei das zweite Antihistaminikum Rupatadin oder ein pharmazeutisch annehmbares Salz davon ist.

12. Verfahren zur Herstellung der Verbindung nach Anspruch 1, umfassend die Umsetzung einer Verbindung der Formel I mit einer Verbindung der Formel III, wobei LG eine Abgangsgruppe ist, in einem Lösungsmittel

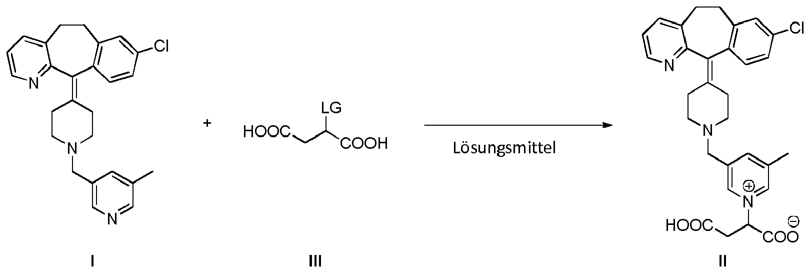

13. Verfahren nach Anspruch 12, wobei das Lösungsmittel aus einem Alkohol, Wasser, Mischungen aus einem Alkohol und Wasser und Mischungen aus Wasser und einem polaren, aprotischen Lösungsmittel. ausgewählt wird

14. Verfahren nach Anspruch 13, wobei das Lösungsmittel ein Alkohol ist.

15. Verfahren zur Herstellung der Verbindung nach Anspruch 1, welches die Umsetzung einer Verbindung der Formel I mit Fumarsäure unter sauren Bedingungen umfasst:

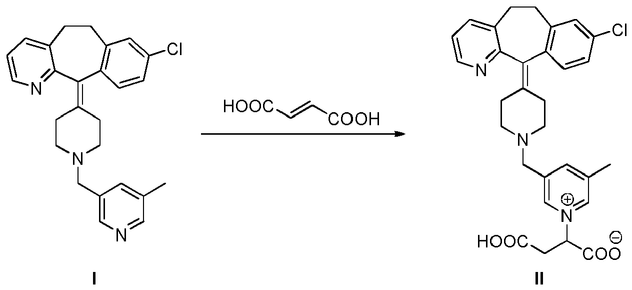

## Revendications

1. Composé de formule II : 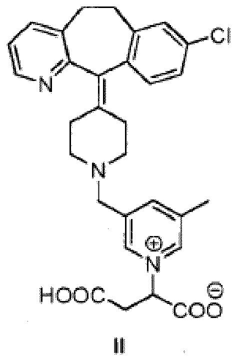 ou un sel pharmaceutiquement acceptable de celui-ci.

2. Composé selon la revendication 1 pour une utilisation en thérapie.

3. Composé selon la revendication 2 pour une utilisation dans le traitement d'un trouble allergique.

4. Composé selon la revendication 2 pour une utilisation dans le traitement de la rhinite allergique ou de l'urticaire.

5. Composition pharmaceutique comprenant le composé selon la revendication 1 et un ou plusieurs excipients pharmaceutiquement acceptables.

6. Composition pharmaceutique selon la revendication 5 qui est une composition pharmaceutique aqueuse liquide.

7. Composition pharmaceutique selon la revendication 6 qui comprend de 0,01 g/l à 10 g/l du composé selon la revendication 1.

8. Composition pharmaceutique selon l'une quelconque des revendications 5 à 7 qui est prévue pour une utilisation orale.

9. Composition pharmaceutique selon l'une quelconque des revendications 5 à 8 qui comprend un second agent antihistaminique.

10. Composition pharmaceutique selon la revendication 9 dans laquelle le second agent antihistaminique est choisi parmi la loratadine, la desloratadine, la rupatadine, la clémastine, la diphénhydramine, la fexofénadine, la phéniramine, la cétirizine, l'ébastine, la prométhazine, la chlorphéniramine et la lévocétirizine, ou un sel pharmaceutiquement acceptable de celles-ci.

11. Composition pharmaceutique selon la revendication 10 dans laquelle le second agent antihistaminique est la rupatadine ou un sel pharmaceutiquement acceptable de celle-ci.

12. Procédé de préparation du composé selon la revendication 1 qui comprend la réaction d'un composé de formule I avec un composé de formule III dans laquelle LG représente un groupe partant, dans un solvant

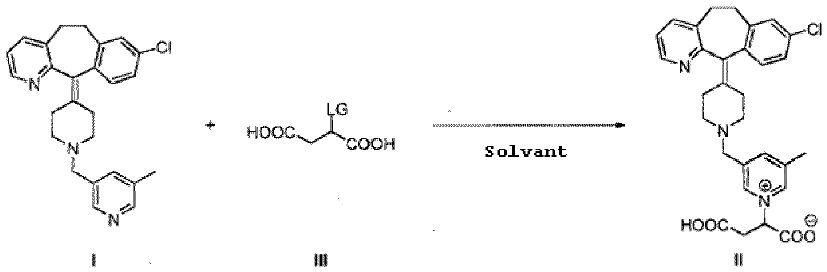

13. Procédé selon la revendication 12 dans lequel le solvant est choisi parmi un alcool, l'eau, des mélanges d'un alcool et d'eau et des mélanges d'eau et d'un solvant polaire aprotique.

14. Procédé selon la revendication 13 dans lequel le solvant est un alcool.

15. Procédé de préparation du composé selon la revendication 1 qui comprend la réaction d'un composé de formule 1 avec de l'acide fumarique dans des conditions acides :
